# EUROPÄISCHE PATENTSCHRIFT

(11) **EP 0 388 732 B1**
(45) Veröffentlichungstag und Bekanntmachung des Hinweises auf die Patenterteilung: **27.04.1994**
(21) Anmeldenummer: 90104509.6
(22) Anmeldetag: 09.03.1990
(51) Int. Cl.: C07C 69/738, C07C 69/734

(54) **Pentasubstituierte Phenyle**
Pentasubstituted phenyls
Phényles pentasubstitués

(30) Priorität: 22.03.1989 DE 3909378
(43) Veröffentlichungstag der Anmeldung: 26.09.1990
(73) Patentinhaber: BAYER AG, 51368 Leverkusen (DE)
(72) Erfinder: Fey, Peter, Dr., D-5600 Wuppertal 1 (DE); Angerbauer, Rolf, Dr., D-5600 Wuppertal 1 (DE); Hübsch, Walter, Dr., D-5600 Wuppertal 1 (DE); Philipps, Thomas, Dr., D-5000 Köln 80 (DE); Bischoff, Hilmar, Dr., D-5600 Wuppertal 1 (DE); Petzinna, Dieter, Dr., D-4000 Düsseldorf 12 (DE); Schmidt, Delf, Dr., D-5600 Wuppertal 1 (DE)

(56) Entgegenhaltungen:
- EP-A- 0 068 038
- EP-A- 0 232 997
- EP-A- 0 344 602
- WO-A-89/05639

## Beschreibung

Die Erfindung betrifft neue pentasubstituierte Phenyle, ihre Herstellung und ihre Verwendung in Arzneimitteln.

Es ist bekannt, daß aus Pilzkulturen isolierte Lactonderivate Inhibitoren der 3-Hydroxy-3-methyl-gluaryl-Coenzym A Reduktase (HMG-CoA-Reduktase) sind [Mevinolin, EP-A 22 478; US 4 231 938]. Darüberhinaus sind auch bestimmte Indolderivate, Pyrazolderivate, Biphenyl- und Phenylderivate Inhibitoren der HMG-CoA-Reduktase [EP-A 1 114 027; US-Patent 4 613 610; US-Patent 4 772 626; EP-A1 028 22 17 und EP-A1 028 32 17].

Die EP 68038 betrifft das tetrasubstituierte Phenylderivat (+)-(4R,6S)-(E)-6-(2-(4'-fluoro-3,3',5-trimethyl-(1,1'-biphenyl)-2-yl)-ethenyl)-3,4,5,6-tetrahydro-4hydroxy-2H-pyran-2-on mit HMG-CoA-Reduktase inhibitorischer Wirkung. In EP 232997 werden ebenfalls tetrasubstituierte Phenylderivate beschrieben, die als Wirkstoffe in Arzneimittel zur Senkung des Blutkollesteringehaltes eingesetzt werden können.

Es wurden nun neue pentasubstituierte Phenyle der allgemeinen Formel (I), 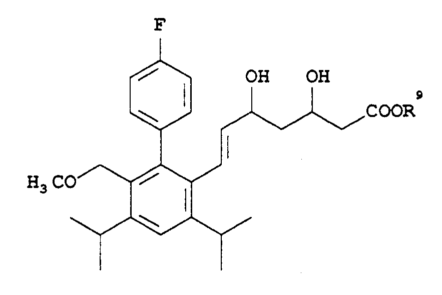
in welcher
- R⁹: für Wasserstoff oder Methyl steht,
und deren Salze gefunden.

Überraschenderweise zeigen die erfindungsgemäßen pentasubstituierten Phenyle eine überlegene inhibitorische Wirkung auf die HMG-CoA-Reduktase (3-Hydroxy-3-methyl-glutaryl-Coenzym A Reduktase).

Die erfindungsgemäßen pentasubstituierten Phenyle der allgemeinen Formel (I) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Bevorzugt sind diejenigen Verbindungen der allgemeinen Formel (I) die E-konfiguriert sind (II).

Bevorzugt sind die Erythro-konfigurierten Isomeren, besonders bevorzugt das 3R,5S-Isomere sowie das 3R,5S-3S, 5R-Racemat.

Außerdem wurde ein Verfahren zur Herstellung der pentasubstituierten Phenyle der allgemeinen Formel (I) 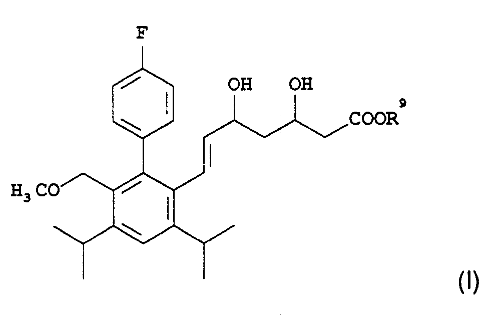
in welcher
- R⁹: für Wasserstoff oder Methyl steht,
und deren Salze gefunden, das dadurch gekennzeichnet ist, daß man Ketone der allgemeinen Formel (II) 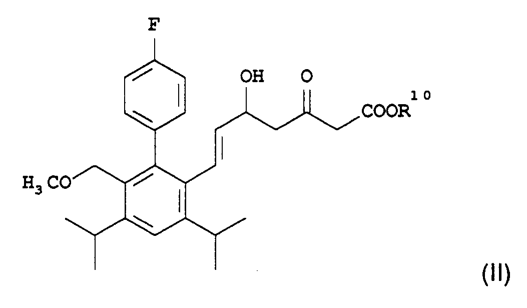
worin R¹⁰ für Alkyl steht, reduziert,
im Fall der Herstellung der Säuren oder Salze die Ester verseift und gegebenenfalls Isomeren trennt.

Das erfindungsgemäße Verfahren kann durch das folgende Formelschema erläutert werden: 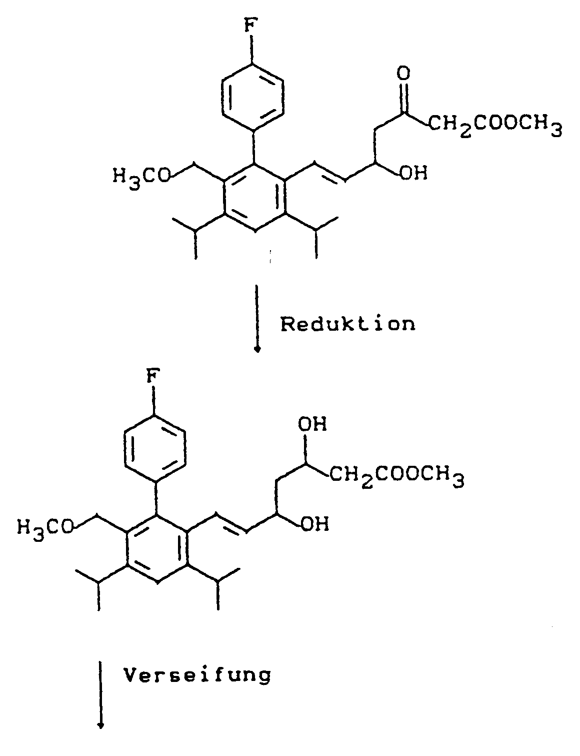 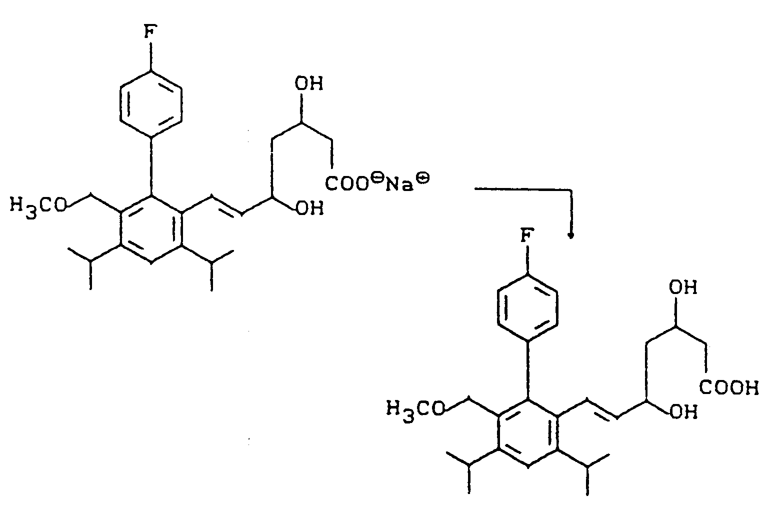
Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-borate, Natrium-trialkyl-hydrido-boranaten, Natrium-cyano-trihydrido-boranat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispielsweise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80°C bis +30°C, bevorzugt von -78°C bis 0°C.

Das erfindungsgemäße Verfahren wird im allgemeinen bei Normaldruck durchgeführt. Es ist aber auch möglich, das Verfahren bei Unterdruck oder bei Überdruck durchzuführen (z. B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Die Carbonsäuren im Rahmen der allgemeinen Formel (I) entsprechen der Formel (Ia) 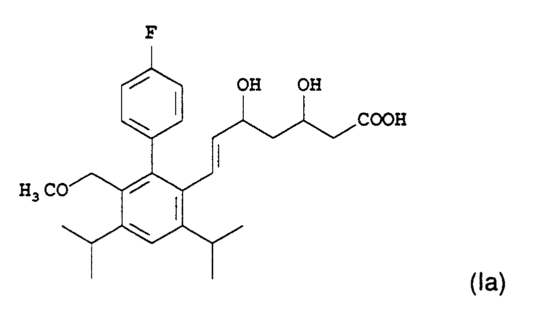
Die Carbonsäureester im Rahmen der allgemeinen Formel (I) entsprechen der Formel (Ib) 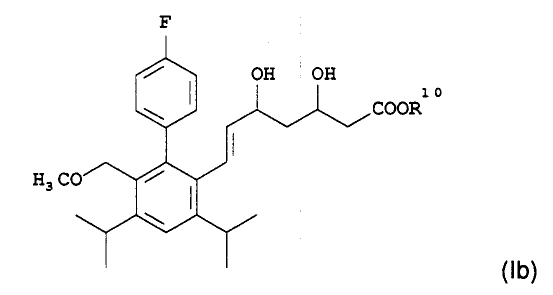
in welcher
- R¹⁰: für Alkyl steht.

Die Salze der erfindungsgemäßen Verbindungen im Rahmen der allgemeinen Formel (I) entsprechen der Formel (Ic) 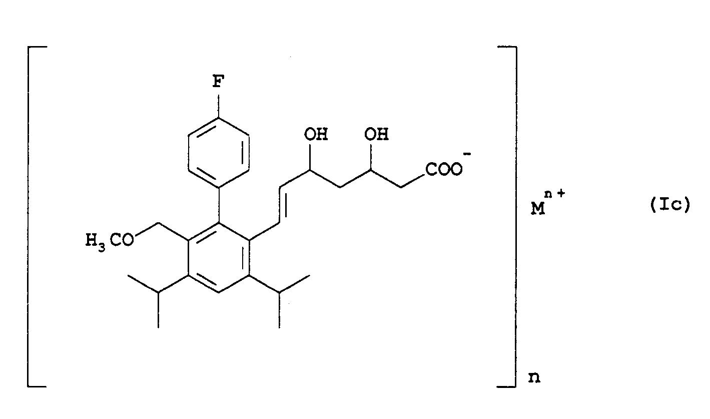
in welcher
- Mⁿ⁺: für ein Kation steht, wobei n die Wertigkeit angibt.

Zur Herstellung der erfindungsgemäßen Carbonsäure (Ia) werden im allgemeinen die Carbonsäureester (Ib) nach üblichen Methoden verseift. Die Verseifung erfolgt im allgemeinen indem man die Ester in inerten Lösemitteln mit üblichen Basen behandelt, wobei im allgemeinen zunächst die Salze der allgemeinen Formel (Ic) entstehen, die anschließend in einem zweiten Schritt durch Behandeln mit Säure in die freien Säuren (Ia) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters eingesetzt. Besonders bevorzugt verwendet man molare Mengen der Reaktanden.

Bei der Durchführung der Reaktion entstehen im ersten Schritt die Salze der erfindungsgemäßen Verbindungen (Ic) als Zwischenprodukte, die isoliert werden können. die erfindungsgemäße Säure (Ia) erhält man durch Behandeln der Salze (Ic) mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäure (Ia) hierbei als vorteilhaft erwiesen, die basische Reaktionsmischung der Verseifung in einem zweiten Schritt ohne Isolierung der Salze anzusäuern. die Säure kann dann in üblicher Weise isoliert werden.

Die Trennung der Isomeren in die stereoisomer einheitlichen Bestandteile erfolgt im allgemeinen nach üblichen Methoden wie sie beispielsweise von E. L. Eliel, Stereochemistry of Carbon Compounds, McGraw Hill, 1962 beschrieben werden. Bevorzugt wird hierbei die Trennung der Isomeren auf der Stufe der racemischen Ester. Besonders bevorzugt wird hierbei die racemische Mischung der trans-Lactone (VII) durch Behandlen entweder mit D-(+)-
oder L-(-)-α-Methylbenzylamin nach üblichen Methoden in die diastereomeren Dihydroxyamide (Ie) 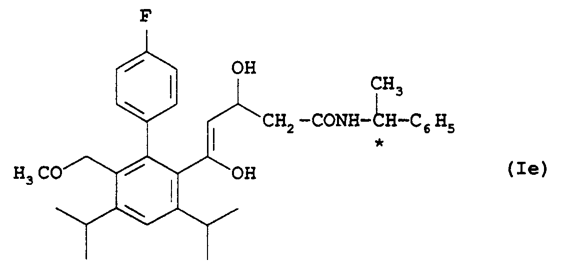
überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kaliumhydroxid in Wasser und/oder organischen Lösemitteln wie Alkoholen z. B. Methanol, Ethanol, Propanol oder Isopropanol, ergeben die entsprechenden enantiomerenreinen Dihydroxysäuren, die wie oben beschrieben durch Cyclisierung in die enantiomerenreinen lactone überführt werden können. Im allgemeinen gilt für die Herstellung der erfindungsgemäßen Verbindungen der allgemeinen formel (I) in eantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden: 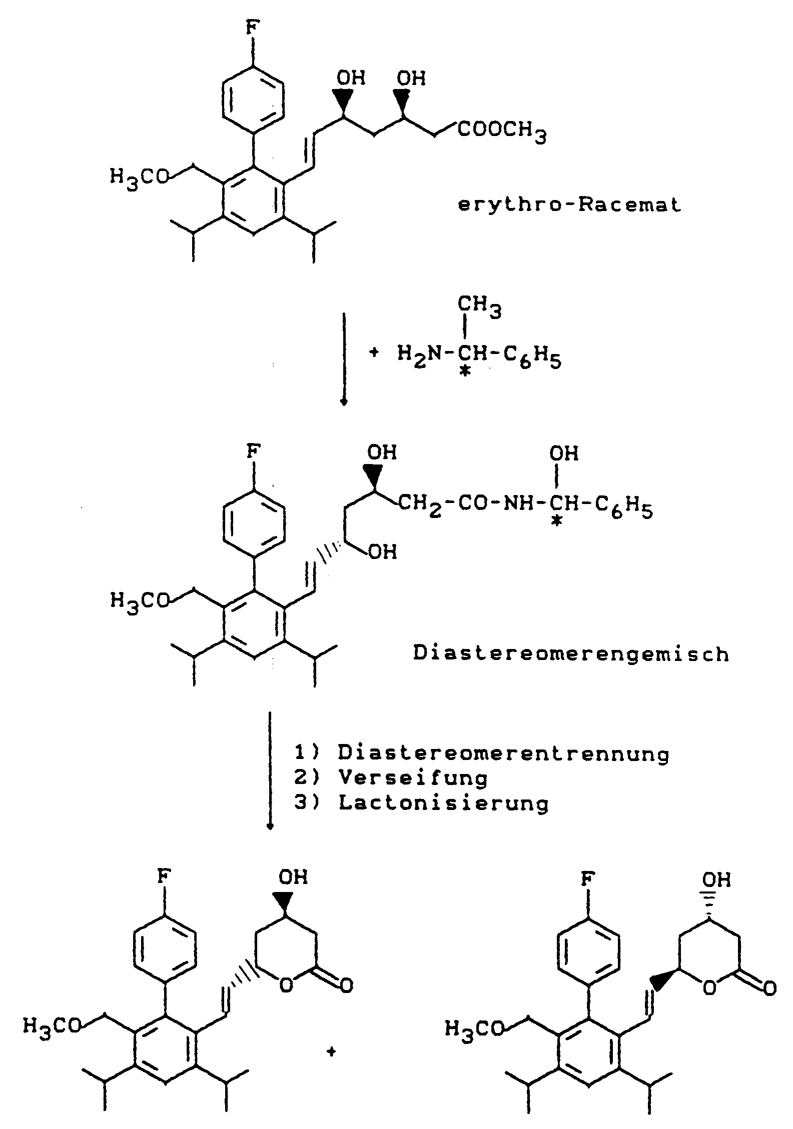
Die als Ausgangsstoffe eingesetzten Ketone (VIII) sind neu.

Es wurde ein Verfahren zur Herstellung der erfindungsgemäßen Ketone der allgemeinen Formel (VIII) 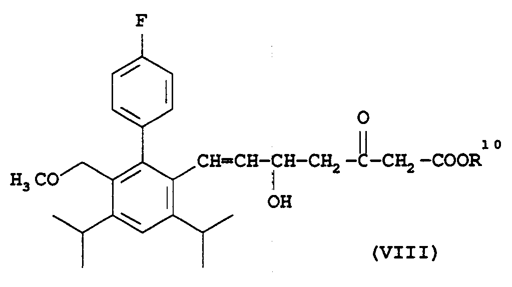
in welcher
- R¹⁰: die oben angegebene Bedeutung hat,
gefunden, das dadurch gekennzeichnet ist, daß man den Aldehyd der Formel (IX) 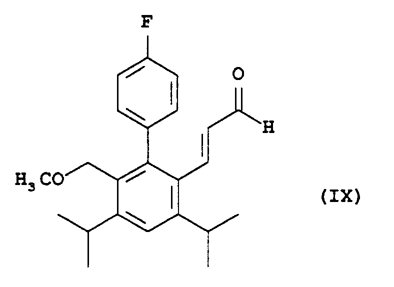
in inerten Lösemitteln mit Acetessigester der allgemeinen Formel (X) 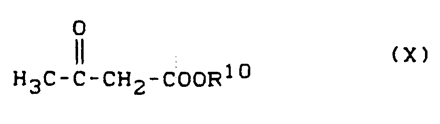
in welcher
- R¹⁰: die oben angegebene Bedeutung hat,
in Anwesenheit von Basen umsetzt.

Das erfindungsgemäße Verfahren kann beispielsweise durch folgendes Formelschema erläutert werden: 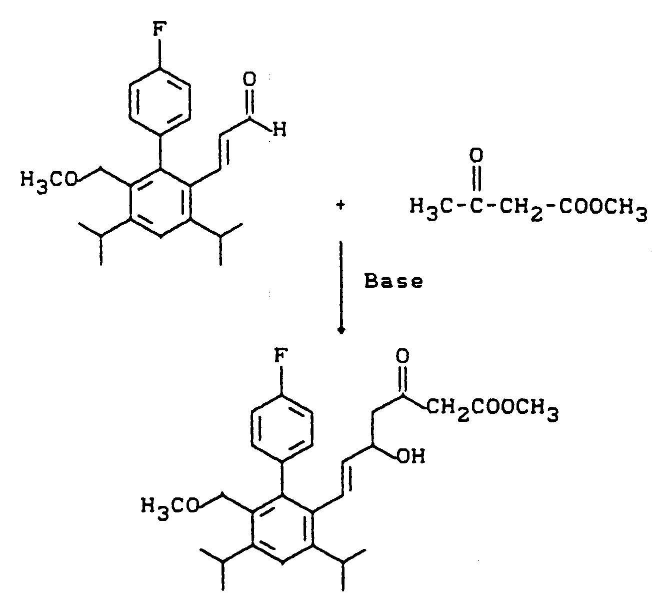
Als Basen kommen hierbei die üblichen stark basischen Verbindungen in Frage. Hierzu gehören bevorzugt lithiumorganische Verbindungen wie beispielsweise n-Butyllithium, sec.Butyllithium, tert.Butyllithium oder Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid oder deren Gemisch eingesetzt.

Eventuell sind Zusätze von Metallhalogeniden wie z.B. Magnesiumchlorid, Zinkchlorid oder Zinkbromid vorteilhaft. Besonders bevorzugt ist der Zusatz von Zinkhalogeniden.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80°C bis +50°C, bevorzugt von -20°C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (XI) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt:
Acetessigsäuremethylester, Acetessigsäureethylester, Acetessigsäurepropylester, Acetessigsäureisopropylester.

Die Herstellung des als Ausgangsstoff eingesetzten Aldehyds der Formel (IX) soll im folgenden beispielhaft für die pentasubstituierten Phenyle erläutert werden. 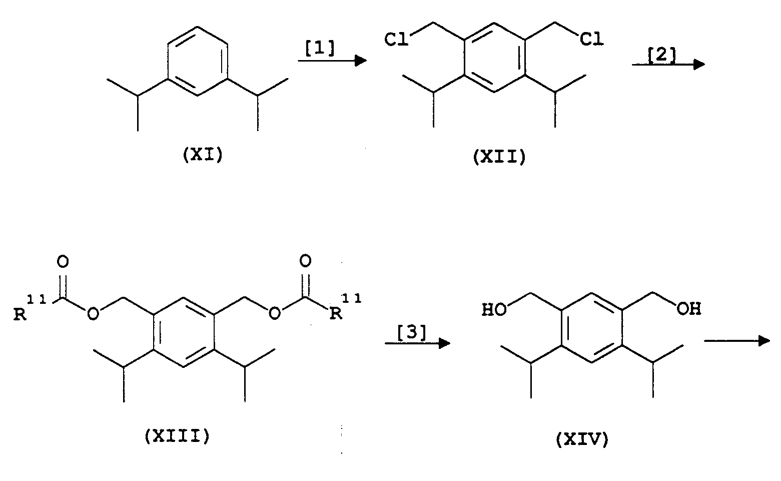 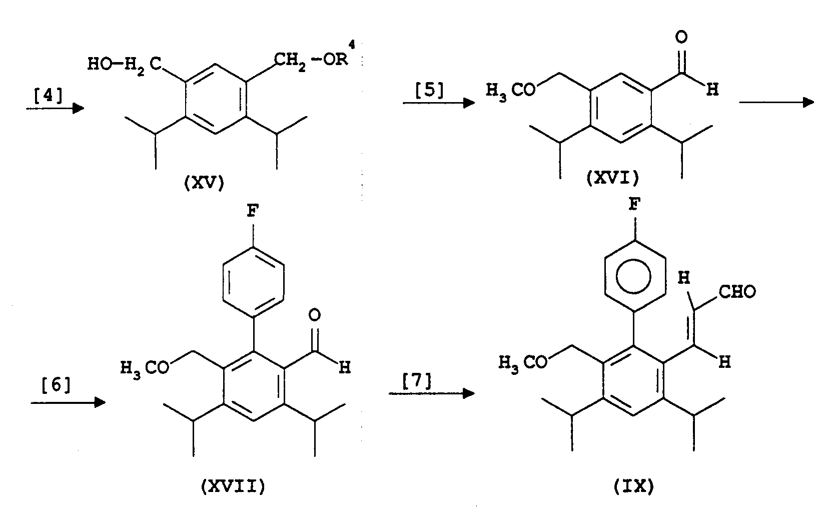
Die Reste A, B, D, E und R⁴ der Formel (XI), (XII), (XIII), (XIV), (XV), (XVI), (XVII) und (IX) haben die oben angegebene Bedeutung und
- R¹¹: steht für einen (C₁-C₄)-Alkylrest.

Hierbei werden substituierte Phenyle der Formel (XI) im ersten Schritt [1] nach bekannten Methoden [Org. React. 1, 68, T. Sato und T. Takemura, J. Chem. Soc., Perk. Trans II, 1195 (1976)] in die Bis-(chlormethyl)-Verbindungen der Formel (XII) überführt. Durch Umsetzung [2] der Verbindungen der Formel (XII) mit z. B. Natriumacetat in Lösemittel wie Dimethylformamid in einem Temperaturbereich von +20°C bis +100°C werden die Bisacetoxy-Verbindung (XIII) erhalten, deren Verseifung [3] nach üblichen Methoden die Bishydroxymethylverbindung (XIV) liefert. In einem vierten Schritt [4] kann die Bishydroxymethylverbindung der Formel (XIV) nach bekannten Methoden z. B. durch Reaktion mit Alkylhalogeniden in Gegenwart von Natrium- oder Kaliumhydrid in den oben erwähnten inerten Lösemitteln wie z. B. Tetrahydrofuran oder durch Reaktion mit Ttialkylchlorsilanen in Gegenwart einer Base wie z. B. Imidazol in Lösemitteln wie z. B. Dimethylformamid, oder durch Acylierung nach üblichen methoden in die Hydroxymethylverbindung (XV) überführt werden. Weiterhin kann eine Hydroxymethylgruppe der Bishydroxymethylverbindungen (XIV) nach bekannten Methoden in eine Abgangsgruppe überführt werden, z. B. durch Umsetzung mit Trifluormethansulfonsäureanhydrid, Thionylchlorid oder Methansulfonsäurechlorid in Gegenwart einer Base. Die Abgangsgruppe kann dann nach bekannten Methoden gegen Nucleophile ausgetauscht werden.

Die Hydroxymethylverbindung (XV) wird im fünften Schritt [5] nach üblichen Methoden zu dem Aldehyd (XVI) oxidiert. Die Oxidation kann beispielsweise mit Pyridiniumchlorochromat, gegebenenfalls in Anwesenheit von Aluminiumoxid, in inerten Lösemitteln wie Chlorkohlenwasserstoffen, vorzugsweise Methylenchlorid, in einem Temperaturbereich von 0°C bis 60°C, bevorzugt bei Raumtemperatur durchgeführt werden, oder aber mit Trifluoressigsäure/Dimethylsulfoxid nach den üblichen Methoden der Swern Oxidation durchgeführt werden. Im sechsten Schritt [6] wird der Aldehyd (XVII) durch Einführung des pro-Substituenten nach bekannter Methode aufgebaut [vgl. Shun-Ichi Murahashi, Yoshihiro Tamba, Masaaki Yamamura und Noriaki Yoshimura, J. Org. Chem. 43, 4099 (1978)]. Der Aldehyd (XVII) wird im siebten Schritt [7] mit Diethyl-2-(cyclohexylamino)-vinylphophonat in Anwesenheit von Natriumhydrid in inerten Lösemitteln wie Ethern, beispielsweise Diethylether, Tetrahydrofuran oder Dioxan, vorzugsweise in Tetrahydrofuran, in einem Temperaturbereich von -20°C bis +40°C, vorzugsweise von -5°C bis Raumteperatur zu dem Aldehyd (IX) umgesetzt.

Die erfindungsgemäßen Verbindungen der allgemeinen Formel (I) besitzen wertvolle pharmakologische Eigenschaften und können in Arzneimitteln eingesetzt werden. Insbesondere sind sie Inhibitoren der 3-Hydroxy-3-methylglutaryl-Coenzym A (HMG-CoA) Reduktase und infolge dessen Inhibitoren der Cholesterolbiosynthese. Sie können deshalb zur Behandlung von Hyperlipoproteinämie, Lipoproteinämie oder Atherosklerose eingesetzt werden. Die erfindungsgemäßen Wirkstoffe bewirken außerdem eine Senkung des Cholesteringehaltes im Blut.

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Cholestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m KₓH_{y} Phosphat, 0,03 m Ethylendiamintetraessig säure, 0,002 m Dithiothreit (SPE)-Puffer pH 7,2, homogenisiert. Anschließend wurde 15 Minuten bei 15 000 g zentrifugiert und das Sediment verworfen. Der Überstand wurde 75 Minuten bei 100 000 g sedimentiert. Das Pellet wird in 1/4 Volumen SPE-Puffer aufgenommen, nochmals homogenisiert und anschließend erneut 60 Minuten bei 100.000 g zentrifugiert. Das Pellet wird mit der 5-fachen Menge ihres Volumes SPE-Puffer aufgenommen, homogenisiert und bei -78°C eingefroren und gelagert (= Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37°C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase 35 µMol KₓH_{y}-Phosphat pH 7,2, 20 µl Enzympräparation und 56 nMol 3xHydroxy-3-methyl-glutaryl Coenzym A (Glutaryl-3-¹⁴C) 100 000 dpm.

Nach Inkubation von 60 Minuten bei 37°C wurde der Ansatz zentrifugiert und 600 µl des Überstandes auf eine 0,7 x 4 cm mit einem 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt und im LKB-Scintillations-zähler gezählt. IC₅₀-Werte wurden durch Auftrag der prozentualen Hemmung gegen die Konzentration der Verbindung im Test durch Intrapolation bestimmt. Zur Bestimmung der relativen inhibitorischen Potenz wurde der IC₅₀-Wert der Referenzsubstanz Mevinolin als 1 gesetzt und mit dem simultan bestimmten IC₅₀-Wert der Testverbindung verglichen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchem die Verabreichung erfolgt.

So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Herstellungsbeispiele

### Beispiel 1

### 2,4-Bis-(chlormethyl)-1,5-diisopropylbenzol

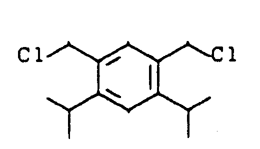

Zu einer Lösung von 230 ml (2,43 mol) Chlormethylethylether und 197 g (1,22 mol) 1,3-Diisopropylbenzol in 600 ml Tetrachlorkohlenstoff tropft man unter Eisbadkühlung (exotherme Reaktion) 55 ml (0,46 mol) Zinntetrachlorid und rührt 1 h bei 0°C. Nach Rühren über Nacht bei +25°C tropft man erneut 142 g (1,5 mol) Chlormethylethylether und 74 ml (0,46 mol) Zinntetrachlorid bei 0°C zu und rührt über Nacht bei +25°C. Das Reaktionsgemisch wird auf Eis/Salzsäure gegeben, die organische Phase abgetrennt, mit Wasser gewaschen und mit Natriumsulfat getrocknet. Nach dem Einengen wird mit Petrolether aufgenommen, über 1 kg Kieselgel (230 - 400 mesh) filtriert und im Hochvakuum destilliert
Sdp.: 133° - 139°C / 1,2 Torr
Ausbeute: 89,9g (28% der Theorie)
¹H-NMR (CDCl₃): δ (ppm) = 1,2 (d, 12H); 3,2 (sept., 2H); 4,55 (s, 4H); 7,2 (2s; 2H).

### Beispiel 2

### 2,4-Bis-(acetoxymethyl)-1,5-diisopropylbenzol

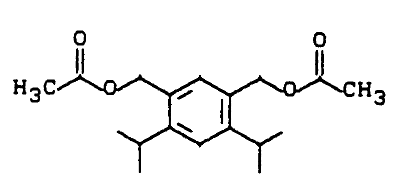

40,0 g (154 mmol) der Verbindung aus Beispiel 1 und 22,7 g (277 mmol) Natriumacetat werden über Nacht in 200 ml Dimethylformamid bei 80°C erhitzt. Nach Abdestillieren des Lösungsmittels wird mit Petrolether aufgenommen, mit Wasser gewaschen und mit Natriumsulfat getrocknet.
Rohausbeute: 40,2 g (85% der Theorie)
¹H-NMR (CDCl₃): δ (ppm) = 1,25 (d, 12H); 2,1 (s, 6H); 3,15 (sept, 2H); 5,15 (s, 4H); 7,3 (2s, 2H).

### Beispiel 3

### 2,4-Bis-(hydroxymethyl)-1,5-diisopropylbenzol

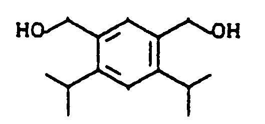

40,2 g (131 mmol) der Verbindung aus Beispiel 2 werden mit 16,1 g (0,3 mol) Kaliumhydroxid in 200 ml Methanol 3 h bei 50°C gerührt. Nach Abdestillieren des Lösungsmittels wird in Ether aufgenommen, mit Wasser gewaschen, mit Natriumsulfat getrocknet und aus Essigester/Petrolether umkristallisiert.
Ausbeute: 17,3 g (59,5% der Theorie)
Schmp.: 102 - 104°C
¹H-NMR (CDCl₃): δ (ppm) = 1,25 (d, 12H); 1,7 (s, 2H); 3,25 (sept., 2H); 4,7 (s, 4H); 7,3 (2s, 2H).

### Beispiel 4

### 2-Hydroxymethyl-4-methoxymethyl-1,5-diisopropylbenzol

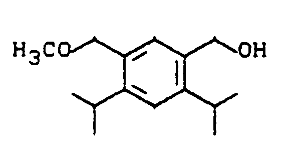

Zu 8,9 g (0,3 mol) Natriumhydrid (80%ig in Paraffinöl) in 500 ml Tetrahydrofuran gibt man unter Stickstoffatmosphäre 31,5 g (0,14 mol) der Verbindung aus Beispiel 3 und 9,6 ml (0,15 mol) Methyliodid. Nach 2 h Rühren bei 80°C wird vorsichtig mit 30 ml verdünnter Salzsäure hydrolysiert, das Lösemittel abdestilliert, in Methylenchlorid aufgenommen, mit Wasser gewaschen, getrocknet und über Kieselgel (230 - 400 mesh, Essigester/Petrolether 1:2) chromatographiert.
Ausbeute: 15,7 g (47,5% der Theorie) und 7,7 g Edukt (24,8% der Theorie)
¹H-NMR (CDCl₃): δ (ppm) = 1,25 (d, 12H); 1,45 (tr, 1H); 3,2 (m, 2H); 3,4 (s, 3H); 4,45 (s, 2H); 4,7 (d, 2H); 7,25 (s, 2H).

### Beispiel 5

### 2,4-Diisopropyl-5-methoxymethyl-benzaldehyd

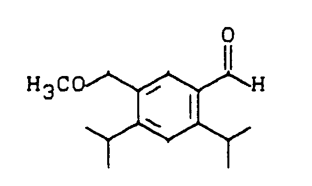

Zu einer Lösung von 15,7 g (66,5 mmol) der Verbindung aus Beispiel 4 in 300 ml Methylenchlorid gibt man 17,2 g (80 mmol) Pyridiniumchlorochromat, rührt über Nacht bei Raumtemperatur, saugt über Kieselgur ab, wäscht mit 200 ml Methylenchlorid nach, und trocknet mit Natriumsulfat.
Ausbeute: 10,4 g (66,8% der Theorie)
¹H-NMR (CDCl₃): δ (ppm) = 1,30 (m, 12H); 3,25 (sept. 1H); 3,4 (s, 3H); 4,0 (sept., 1H); 4,56 (s, 2H); 7,4 (s, 1H); 7,75 (s, 1H); 10,3 (s, 1H).

### Beispiel 6

### N-[2,4-Diisopropyl-5-methoxymethyl-benzal]-phenylamin

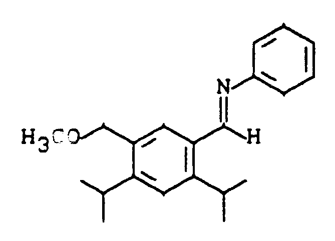

Eine Lösung von 10,36 g (44,3 mmol) der Verbindung aus Beispiel 5, 4,06 g (44,6 mmol) frisch destilliertes Anilin und 0,3 g p-Toluolsulfonsäure in 370 ml Toluol wird 1,5 h am Wasserabscheider (Molekularsieb 4Å) unter Rückfluß erhitzt, nach Abkühlen auf Raumtemperatur mit Essigester verdünnt, mit gesättigter Natriumhydrogencarbonatlösung und wasser gewaschen, mit Natriumsulfat getrocknet und im Vakuum eingeengt.
Ausbeute: 13,26 g Öl (98,5% der Theorie)
¹H-NMR (CDCl₃): δ (ppm) = 1,3 (m, 12H); 3,3 (sept., 1H); 3,4 (s, 3H); 3,65 (sept, 1H); 4,55 (s, 2H); 7,1 - 7,4 (m, 7H); 8,0 (s, 1H); 8,8 (s, 1H).

### Beispiel 7

### Bis{2,4-diisopropyl-5-methoxymethyl-1-[(phenylimino)methyl]phenyl-C,N}-dipalladium

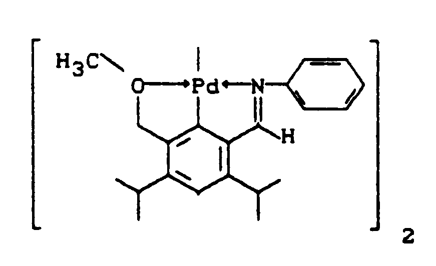

Eine Mischung von 13,25 g (42,75 mmol) der Verbindung aus Beispiel 6 und 9,60 g (42,75 mmol) Palladium-(II)-acetat in 200 ml Eisessig wird 1 h unter Rückfluß erhitzt. Nach Abkühlen auf Raumtemperatur wird das Reaktionsgemisch auf 800 ml Wasser gegossen, der ausgefallene Feststoff abgesaugt und mit Wasser gewaschen. Die wässrige Lösung wird zur Trockne eingeengt, in Essigester aufgenommen, mit Natriumhydrogencarbonatlösung und Natriumchloridlösung gewaschen, mit Natriumsulfat getrocknet und nach dem Einengen mit 100 ml Petrolether/Essigester 1:1 verrührt. Das ausgefallene Produkt wird im Vakuum über Kaliumhydroxid getrocknet.
Ausbeute: 7,1 g (39% der Theorie)
¹H-NMR (CDCl₃): δ (ppm) = 1,3 (m, 12H); 2,7 (sept, 1H); 3,2 (sept, 1H); 3,95 (s, 3H); 5,8 (s, 2H); 6,85 (s, 1H); 7,25 - 7,6 (m, 5H); 8,15 (s, 1H).

### Beispiel 8

### 1,1′-Biphenyl-3,5-diisopropyl-4′-fluoro-6-methoxymethyl-2-carbaldehyd

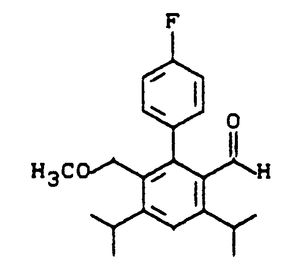

Eine Lösung von 3,5 g (4,1 mmol) der Verbindung aus Beispiel 7 und 8,35 g (31,9 mmol) Triphenylphosphin in 80 ml Benzol wird 30 min bei Raumtemperatur unter Stickstoffatmosphäre gerührt. Zu dieser Lösung wird eine Lösung von 4-Fluorphenylmagnesiumbromid (hergestellt aus 0,845 g (34,7 mmol) Magnesium und 3,94 ml (35,2 mmol) 4-Bromfluorbenzol in 30 ml Ether) gegeben und über Nacht gerührt. Unter Eiskühlung wird mit 24 ml 6 N Salzsäure hydrolysiert, 1 h bei Raumtemperatur gerührt, der ausgefallene Feststoff abgesaugt, das Filtrat mit Ether verdünnt, mit Natriumchloridlösung gewaschen und mit Natriumsulfat getrocknet. Nach Einengen erhält man 11,21 g Öl. Die Chromatographie über Kieselgel (230 - 400 mesh, Essigester/Petrolether 1:20) ergibt 1,05 g Rohprodukt, erneute Chromatographie über Kieselgel (70 - 230 mesh, Essigester/Petrolether 1:80) ergibt 670 mg Feststoff.
Ausbeute: 50% der Theorie
Schmpkt.: 62 - 70°C
¹H-NMR (CDCl₃): δ (ppm) = 1,3 (m, 12H); 3,15 (s, 3H); 3,35 (sept, 1H); 3,9 (sept, 1H); 4,05 (s, 2H); 7,05 - 7,3 (m, 4H); 7,45 (s, 1H); 9,7 (s, 1H).

### Beispiel 9

### (E)-3-[1,1′-Biphenyl-3,5-diisopropyl-4′-fluoro-6-methoxymethyl-2-yl]-prop-2-enal

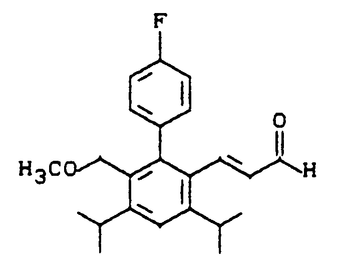

Unter Stickstoff tropft man zu einer Suspension von 53 mg (2,23 mmol) Natriumhydrid in 6 ml trockenem Tetrahydrofuran bei -5°C 728 mg (2,79 mmol) Diethyl-2-(cyclohexylamino)-vinylphosphonat, gelöst in 6 ml trockenem Tetrahydrofuran. Nach 30 min werden bei derselben Temperatur 610 mg (1,86 mmol) der Verbindung aus Beispiel 8 in 15 ml trockenem Tetrahydrofuran zugetropft und 30 min zum Rückfluß erwärmt. Nach Abkühlen auf Raumtemperatur wird der Ansatz in 200 ml eiskaltes Wasser gegeben und dreimal mit je 100 ml Essigester extrahiert. Die vereinigten organischen Phasen werden mit gesättigter Natriumchlorid-Lösung gewaschen und über Natriumsulfat getrocknet. Nach Einengen im Vakuum wird der Rückstand in 5 ml Toluol aufgenommen, mit einer Lösung von 0,9 g (7 mol) Oxalsäure-Dihydrat in 12 ml Wasser versetzt und 90 min zum Rückfluß erhitzt. Nach Abkühlen auf Raumtemperatur werden die Phasen getrennt, die organische Phase mit gesättigter Natriumchlorid-Lösung gewaschen, über Natriumsulfat getrocknet und im Vakuum eingeengt. Der Rückstand wird über Kieselgel (230 - 400 mesh, Essigester/Petrolether 1:10) chromatographiert.
Ausbeute: 480 mg (72,8% der Theorie)
Schmpt.: 94 - 96°C
¹H-NMR (CDCl₃): δ (ppm) = 1,25 (d, 6H); 1,35 (d, 6H); 3,15 (s, 3H); 3,2 (sept., 1H); 3,35 (sept., 1H); 4.05 (s, 2H); 5,95 (dd, 1H); 7,0 - 7,3 (m, 5H); 7,4 (s, 1H); 9,35 (d, 1H).

### Beispiel 10

### Methyl-(E)-7-[1,1′-biphenyl-3,5-diisopropyl-4′-fluoro-6-methoxymethyl-2-yl]-5-hydroxy-3-oxo-hept-6-enoat

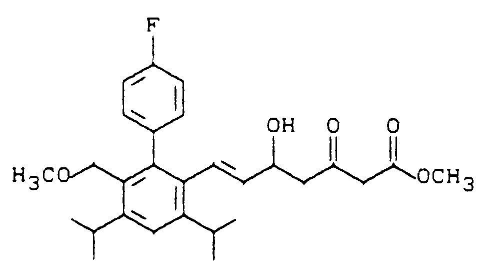

Unter Stickstoff tropft man zu einer Suspension von 70 mg (2,92 mmol) Natriumhydrid in 3 ml trockenem Tetrahydrofuran bei -5°C 0,31 ml (2,84 mmol) Acetessigsäuremethylester. Nach 15 min werden bei derselben Temperatur 1,81 ml (2,89 mmol) 15%iges n-Butyllithium in n-Hexan zugetropft und 15 min nachgerührt. Anschließend werden 480 mg (1,35 mmol) der Verbindung aus Beispiel 9 gelöst in 8 ml trockenem Tetrahydrofuran zugetropft und 30 min bei -5°C nachgerührt. Die Reaktionslösung wird vorsichtig mit 100 ml gesättigter wässriger Ammoniumchloridlösung verdünnt und die Mischung dreimal mit je 100 ml Ether extrahiert. Die vereinigten organischen Phasen werden zweimal mit gesättigter Natriumhydrogencarbonat-Lösung und einmal mit gesättigter Natriumchlorid-Lösung gewaschen, über Natriumsulfat getrocknet und im Vakuum eingeengt.
Rohausbeute: 740 mg (100% der Theorie)
¹H-NMR (CDCl₃): δ (ppm) = 1,25 (m, 12H); 2,4 (m, 2H); 3,15 (s, 3H); 3,3 (m, 2H); 3,45 (s, 2H); 3,75 (s, 3H); 4,05 (s, 2H); 4,45 (m, 1H); 5,2 (dd, 1H); 6,35 (d, 1H); 7,0 - 7,25 (m, 4H); 7,3 (s, 1H).

### Beispiel 11

### Methyl-erythro-(E)-7-[1,1′-biphenyl-3,5-diisopropyl-4′-fluoro-6-methoxymethyl-2-yl]-3,5-dihydroxy-hept-6-enoat

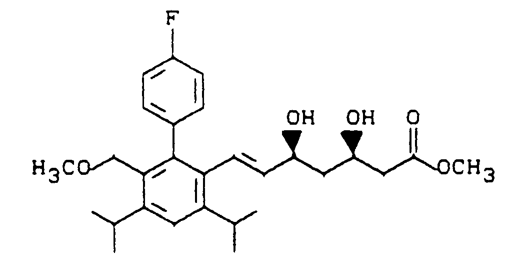

Zu einer Lösung von 680 mg (1,45 mmol) der Verbindung aus Beispiel 10 in 13 ml trockenem Tetrahydrofuran gibt man bei Raumtemperatur 1,7 ml (1,7 mmol) 1 M Triethylboran-Lösung in Tetrahydrofuran, leitet während 5 min Luft durch die Lösung und kühlt auf -30°C Innentemperatur ab. Es werden 66 mg (1,7 mmol) Natriumborhydrid und langsam 1,2 ml Methanol zugegeben, 30 min bei -30°C gerührt und dann mit einem Gemisch von 4,7 ml 30%igem Wasserstoffperoxid und 10 ml Wasser versetzt. Die Temperatur läßt man dabei bis 0°C ansteigen und rührt noch 30 min nach. Die Mischung wird dreimal mit je 70 ml Essigester extrahiert, die vereinigten organischen Phasen je einmal mit 10%iger Kaliumjodidlösung, 10%iger Natriumthiosulfatlösung, gesättigter Natriumhydrogencarbonat-Lösung und gesättigter Natriumchlorid-Lösung gewaschen, über Natriumsulfat getrocknet und im Vakuum eingeengt. Der Rückstand wird an einer Säule (Kieselgel 230 - 400 mesh, Essigester/Petrolether 1:2) chromatographiert.
Ausbeute: 480 mg (70,5% der Theorie)
¹H-NMR (CDCl₃): δ (ppm) = 1,25 (m, 12H); 1,4 (m, 2H); 2,4 (m, 2H); 3,1 (s, 3H); 3,15 (m, 1H); 3,3 (m, 1H); 3,7 (s, 3H); 4,05 (m, 3H); 4,25 (m, 1H); 5,2 (dd, 1H); 6,3 (d, 1H); 6,95 - 7,15 (m, 4H); 7,3 (s, 1H).

## Patentansprüche (Patentansprüche für folgende(n) Vertragsstaat(en): AT, BE, CH, DE, DK, FR, GB, GR, IT, LI, LU, NL, SE)

1. Pentasubstituiertes Phenyl der Formel 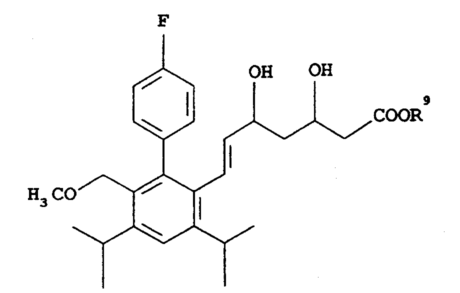 in welcher
R⁹ für Wasserstoff oder Methyl steht,
und deren Salze.

2. Pentasubstituierte Phenyl nach Anspruch 1 zur therapeutischen Behandlung.

3. Verfahren zur Herstellung von pentasubstituierten Phenylen nach Anspruch 1, dadurch gekennzeichnet, daß man Ketone der allgemeinen Formel 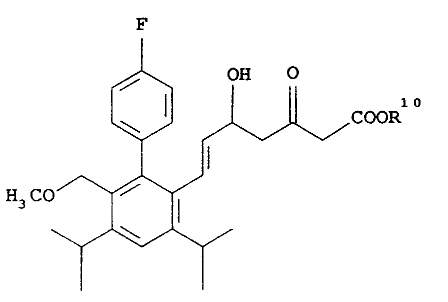 worin R¹⁰ für Alkyl steht, reduziert,
im Fall der Herstellung der Säuren oder Salze die Ester verseift und gegebenenfalls Isomeren trennt.

4. Arzneimittel enthaltend mindestens 1 pentasubstituiertes Phenyl nach Anspruch 1.

5. Arzneimittel nach Anspruch 4 zur Behandlung von Lipoproteinämie und Atherosklerose.

6. Verfahren zur Herstellung von Arzneimitteln nach Anspruch 4 und 5, dadurch gekennzeichnet, daß man die pentasubstituierten Phenyle gegebenenfalls mit üblichen Hilfs- und Trägerstoffen in eine geeignete Applikationsform überführt.

7. Verwendung der pentasubstituierten Phenyle nach Anspruch 1 zur Herstellung von Arzneimitteln.

## Patentansprüche (Patentansprüche für folgende(n) Vertragsstaat(en): ES)

1. Verfahren zur Herstellung von pentasubstituierten Phenylen der allgemeinen Formel 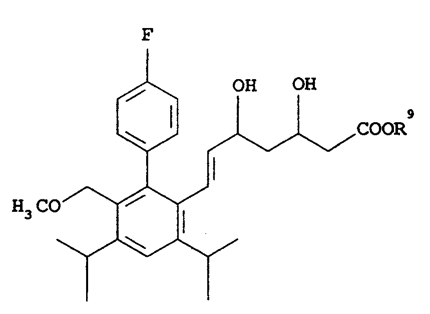 in welcher
R⁹ für Wasserstoff oder Methyl steht,
und deren Salze, dadurch gekennzeichnet, daß man
Ketone der allgemeinen Formel 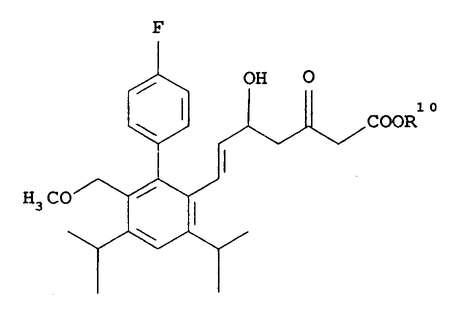 worin R¹⁰ für Alkyl steht, reduziert,
im Fall der Herstellung der Säuren oder Salze die Ester verseift und gegebenenfalls Isomeren trennt.

2. Verwendung der nach Anspruch 1 hergestellten pentasubstituierten Phenyle zur Herstellung von Arzneimitteln.

## Claims (Claims for the following Contracting State(s): AT, BE, CH, DE, DK, FR, GB, GR, IT, LI, LU, NL, SE)

1. Pentasubstituted phenyl of the formula 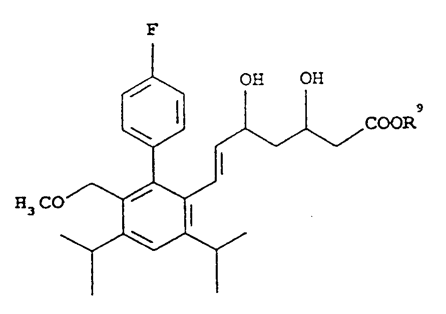 in which
R⁹ represents hydrogen or methyl,
and their salts.

2. Pentasubstituted phenyl according to Claim 1 for therapeutic treatment.

3. Process for the preparation of pentasubstituted phenyl according to Claim 1, characterized in that ketones of the general formula 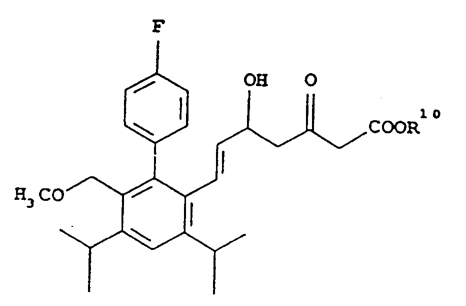 in which R¹⁰ represents alkyl, are reduced,
in the case of the preparation of the acids or salts, the esters are hydrolysed and, if appropriate, isomers are separated.

4. Medicaments, containing at least 1 pentasubstituted phenyl according to Claim 1.

5. Medicaments according to Claim 4 for the treatment of lipoproteinaemia and atherosclerosis.

6. Process for the preparation of medicaments according to Claims 4 and 5, characterized in that pentasubstituted phenyls are brought into a suitable form for administration, if appropriate using customary auxiliaries and excipients.

7. Use of the pentasubstituted phenyls according to Claim 1 for the preparation of medicaments.

## Claims (Claims for the following Contracting State(s): ES)

1. Process for the preparation of pentasubstituted phenyls of the general formula 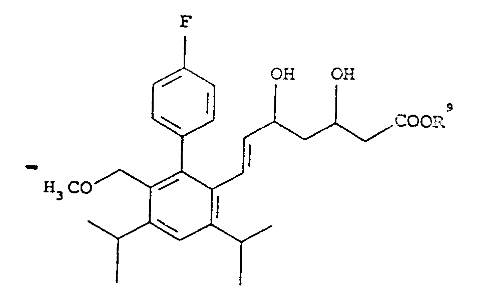 in which
R⁹ represents hydrogen or methyl,
and their salts, characterized in that ketones of the general formula 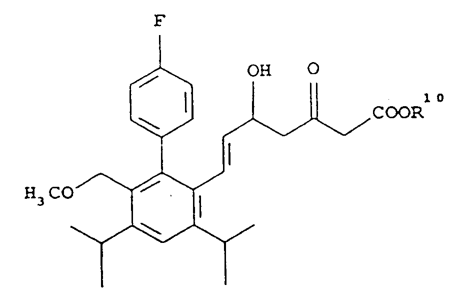 in which R¹⁰ represents alkyl, are reduced,
in the case of the preparation of the acids or salts, the eaters are hydrolysed and, if appropriate, isomers are separated.

2. Use of the pentasubstituted phenyls prepared according to Claim 1 for the preparation of medicaments.

## Revendications (Revendications pour l'(les) Etat(s) contractant(s) suivant(s): AT, BE, CH, DE, DK, FR, GB, GR, IT, LI, LU, NL, SE)

1. Phényle pentasubstitué répondant à la formule 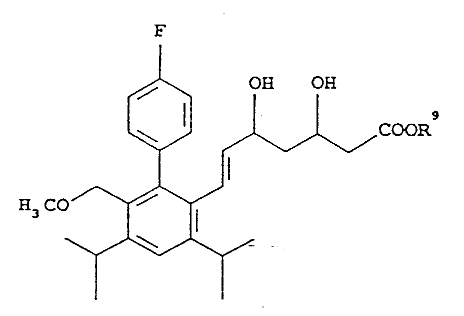 dans laquelle
R⁹ représente un atome d'hydrogène ou un groupe méthyle,
ainsi que ses sels.

2. Phényles pentasubstitués selon la revendication 1, pour le traitement thérapeutique.

3. Procédé pour la préparation de phényles pentasubstitués selon la revendication 1, caractérisé en ce qu'on réduit des cétones répondant à la formule générale 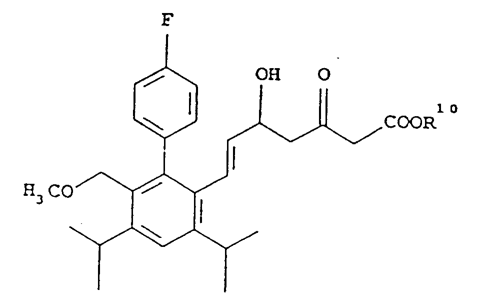 dans laquelle R¹⁰ représente un groupe alkyle,
en cas de préparation des acides ou des sels, on saponifie les esters et éventuellement on sépare les isomères.

4. Médicament contenant au moins un phényle pentasubstitué selon la revendication 1.

5. Médicament selon la revendication 4, pour le traitement de la lipoprotéinémie et de l'athérosclérose.

6. Procédé pour la préparation de médicaments selon les revendications 4 et 5, caractérisé en ce qu'on transforme les phényles pentasubstitués éventuellement avec des substances auxiliaires et de support habituelles en une forme d'application appropriée.

7. Utilisation des phényles pentasubstitués selon la revendication 1, pour la préparation de médicaments.

## Revendications (Revendications pour l'(les) Etat(s) contractant(s) suivant(s): ES)

1. Procédé pour la préparation de phényles pentasubstitués répondant à la formule générale 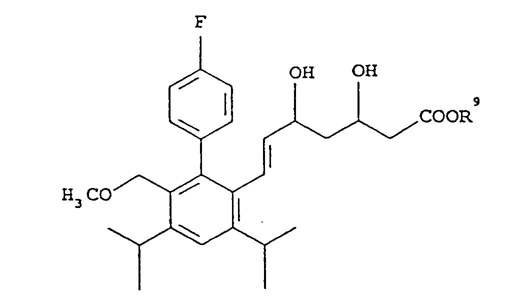 dans laquelle
R⁹ représente un atome d'hydrogène ou un groupe méthyle,
et de leurs sels, caractérisé en ce qu'on réduit
des cétones répondant à la formule générale 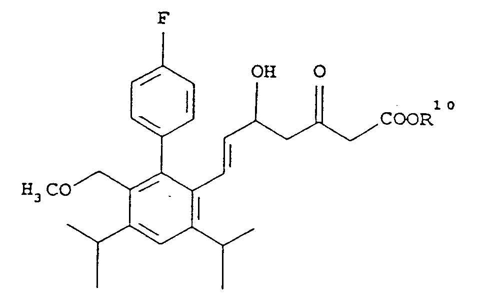 dans laquelle R¹⁰ représente un groupe alkyle,
en cas de préparation des acides ou des sels, on saponifie les esters et éventuellement on sépare les isomères.

2. Utilisation des phényles pentasubstitués préparés conformément à la revendication 1, pour la préparation de médicaments.
